# FASCICULE DE BREVET EUROPEEN

(11) **EP 3 548 099 B1**
(45) Date de publication et mention de la délivrance du brevet: **30.11.2022**
(21) Numéro de dépôt: 17808054.5
(22) Date de dépôt: 27.11.2017
(51) Int. Cl.: A61L 2/06, B27K 5/00

(54) **ETUVE POUR L'ELIMINATION EN CONTINU DE NUISANCES PHYTOSANITAIRES PRESENTES DANS DES PARTICULES ORGANIQUES D'ORIGINE VEGETALE**
OFEN ZUR KONTINUIERLICHEN ENTFERNUNG VON PHYTOSANITÄREN SCHÄDLINGEN IN ORGANISCHEN PARTIKELN PFLANZLICHEN URSPRUNGS
OVEN FOR CONTINUOUS ELIMINATION OF PHYTOSANITARY PESTS PRESENT IN ORGANIC PARTICLES OF PLANT ORIGIN

(30) Priorité: 29.11.2016 BE 201605886
(43) Date de publication de la demande: 09.10.2019
(73) Titulaire: Crosset, Léon, 4890 Thimister (BE)
(72) Inventeur: Crosset, Léon, 4890 Thimister (BE)
(74) Mandataire: Connor, Marco Tom
(86) Numéro de dépôt international: PCT/EP2017/080493
(87) Numéro de publication internationale: WO 2018/099850

(56) Documents cités:
- EP-A1- 0 197 171
- EP-A1- 1 224 946
- WO-A1-2013/139720
- BE-A5- 1 020 153

## Description

### DOMAINE DE L'INVENTION

L'invention se rapporte à une étuve permettant l'élimination des organismes nuisibles présentant des risques phytosanitaires, telles des insectes ou autre organismes présents dans des particules, telles que des copeaux ou sciures de bois. En particulier, la présente étuve remplit les exigences phytosanitaires s'appliquant généralement à l'importation de sciures ou copeaux de bois et d'écorce, et autres sous-produits de bois sous forme de particules, ainsi que de cônes secs destinés à l'importation vers l'Europe ou à l'exportation hors d'Europe. La présente étuve permet de traiter de tels matériaux en continu et de manière efficace d'un point de vue énergétique.

### ARRIERE-PLAN TECHNOLOGIQUE

Dans un environnement de commerce globalisé, le transport à travers les frontières de matériaux d'origine végétale, en particulier du bois sous toutes ses formes, engendre un risque de dissémination d'organismes nuisibles présents dans lesdits matériaux. Ce risque concerne par exemple les emballages et palettes en bois, mais il concerne aussi du bois ou autres matériaux organiques d'origine végétale sous forme de particules, telles que des sciures, des copeaux, des pellets, laine de bois, des morceaux d'écorces, des cônes, etc.

Afin d'apporter une parade efficace au risque de dissémination des organismes nuisibles, tout en évitant au maximum les risques d'entrave aux échanges internationaux, des normes internationales ont été instituées imposant un traitement insecticide aux bois circulant entre différents pays. Par exemple, de plus en plus de pays appliquent la norme internationale pour les mesures phytosanitaires n° 15 de l'Organisation des Nations unies pour l'alimentation et l'agriculture (FAO) relative aux « Directives pour la réglementation de matériaux d'emballage à base de bois dans le commerce international » (NIMP 15) pour éviter la dissémination de parasites du bois. L'importation de marchandises dans ces pays doit se faire avec des emballages en bois (caisses, palettes, etc.) qui ont été soumis à un traitement phytosanitaire strict. En particulier, la norme exige que le bois d'emballage soit traité selon un traitement thermique chauffant le bois à une température centrale minimale de 56°C pendant 30 minutes au moins.

Bien que la norme NIMP 15 (ISMP 15 en anglais) ne soit valable que pour le bois massif, un traitement thermique identique est demandé aussi pour des importations de particules de bois et autres particules d'origine végétale par de nombreux pays.

Un traitement thermique portant le cœur des particules à une température de traitement, Tt, par exemple, de 56°C et la maintenir pendant un temps, t1, par exemple, de 30 min représente un défi technique et économique pour l'exportateur. Un tel traitement thermique peut bien entendu être appliqué par batch, en traitant un volume donné de particules d'origine végétales dans une étuve ou four de capacité adaptée. Cependant, un tel procédé est long et requiert de nombreuses manipulations pour charger le matériau à traiter dans l'étuve ; attendre qu'il atteigne la température, Tt ; maintenir le matériau à la température, Tt, pendant un temps, t1 ; et décharger le matériau traité hors de l'étuve. A la fin de ce procédé, on peut recharger un nouveau volume de matériau à traiter et reproduire le cycle décrit ci-dessus. Une telle solution n'est pas satisfaisante.

Il existe des sécheurs de particules fonctionnant en continu, tel que par exemple le sécheur décrit dans WO2013139720A1, ou EP0197171, qui permettent de sécher des particules dans des conditions de temps et énergie très avantageuses. Cependant, sécher des particules consiste à éliminer l'eau imprégnée dans les particules, ce qui n'a pas grand-chose à voir avec un traitement thermique portant ces mêmes particules à une température, Tt, et les maintenant à cette température pendant un temps, t1. L'utilisation d'un sécheur continu n'est donc pas idéale pour le traitement phytosanitaire de particules de bois et d'autres matériaux d'origine végétale.

La présente invention propose une étuve particulièrement adaptée au traitement en continu de particules de bois et autres matériaux d'origine végétale satisfaisant aux normes généralement appliquées aux exportations de tels produits. L'étuve de la présente invention permet d'assurer une température, Tt, et un temps, t1, de traitement des particules dans un procédé efficace en temps, énergétiquement optimisé et n'occupant qu'une surface au sol limitée. L'étuve de la présente invention est de plus facile d'entretien et économique. Ces avantages et d'autres sont décrits plus en détails dans la Description Détaillée qui suit.

### RESUME DE L'INVENTION

La présente invention est définie dans les revendications indépendantes. Des variantes préférées sont définies dans les revendications dépendantes. En particulier, la présente invention concerne une étuve pour l'élimination d'organismes nuisibles présentant des risques phytosanitaires présents dans des matériaux d'origine végétale sous forme de particules, ladite étuve comprenant,
(a) Une enceinte comprenant une paroi essentiellement cylindrique s'étendant le long d'un axe vertical, Z,
(b) Un premier plateau circulaire monté sur la paroi de ladite enceinte sensiblement normal à l'axe vertical, Z, et agencé pour tourner à une première vitesse de rotation, v1, dans un premier sens autour de l'axe vertical, Z, la surface dudit plateau étant perforée, et perméable à l'air, à la vapeur d'eau et à l'eau,
(c) Un second plateau circulaire monté à une certaine distance du premier plateau sur la paroi de ladite enceinte sensiblement normal à l'axe vertical, Z, et agencé pour tourner à une seconde vitesse de rotation, v2, autour dudit axe vertical, Z, de préférence dans le sens inverse de rotation du premier plateau, la surface dudit plateau étant perforée et perméable à l'air, à la vapeur d'eau et à l'eau,
(d) Un premier moyen de répartition desdites particules apte à répartir lesdites particules avant étuvage le long d'un rayon du premier plateau,
(e) Un premier moyen de récupération des particules réparties sur le premier plateau après une rotation d'un angle donné de celui-ci, le dit premier moyen de récupération étant situé en aval du, et préférablement adjacent au premier moyen de répartition,
(f) Un moyen de transfert des particules récoltées du premier plateau par le premier moyen de récupération vers un second moyen de répartition apte à répartir lesdites particules le long d'un rayon du second plateau, et
(g) Un moyen de soufflage de gaz formant un cycle gazeux fermé, comprenant :
   - une soufflerie pour donner une vitesse à un flux de gaz et le diriger vers,
   - une station de chauffage pour former un flux de gaz chaud ayant une température initiale, T0, et une humidité relative initiale, RH0, et ensuite le diriger vers,
   - un déflecteur amont, déviant le flux de gaz chaud suivant un flux sensiblement parallèle à l'axe Z, ayant une première température, T1, et une première humidité relative, RH1, passant d'abord à travers la surface perforée du premier plateau, où il perd de l'énergie calorifique et d'où en ressort un flux de gaz refroidi ayant une seconde température, T2, et une seconde humidité relative, RH2, pour ensuite passer directement après à travers la surface perforée du second plateau, où il perd encore de l'énergie calorifique et d'où en ressort un flux de gaz froid ayant une troisième température, T3, et une troisième humidité relative, RH3, pour ensuite atteindre,
   - un déflecteur aval déviant le flux de gaz froid vers la soufflerie et recommencer le cycle gazeux.

Dans une première variante de l'invention, le premier plateau est situé en dessus du second plateau et le gaz chaud circule du haut vers le bas et est préférablement de l'air chaud. Dans une deuxième variante de l'invention, le premier plateau est situé au-dessous du second plateau et le gaz chaud circule du bas vers le haut et est également de préférence de l'air chaud.

L'étuve peut comprendre un contrôleur configuré pour contrôler que la première vitesse de rotation, v1, du premier plateau soit de préférence supérieure à la seconde vitesse de rotation, v2, du second plateau. Par exemple, v2 = 1/k · v1, dans lequel, |k| ≥ 1, et la valeur absolue de k est de préférence compris entre 1 et 5, de préférence entre 2 et 4, encore de préférence, |kl = 3, et dans lequel v2 est de préférence compris entre 0.5 et 1.2 tours par heure.

L'étuve peut comprendre un contrôleur configuré pour contrôler les températures et humidités relatives des flux de gaz. Afin d'efficacement éliminer les nuisances phytosanitaires, la première température, T1, du flux de gaz chaud (52) est de préférence comprise entre 75 et 120°C, de préférence entre 85 et 100°C, encore de préférence entre 90 et 95°C. La première humidité relative, RH1 dudit flux de gaz chaud est de préférence comprise entre 15 et 60%, de préférence ≥ 20%. La seconde température, T2, du flux de gaz refroidi est de préférence comprise entre 60 et 80°C, de préférence entre 65 et 70°C, avec une valeur de la seconde humidité relative, RH2 dudit flux de gaz refroidi de préférence comprise entre 60 et 90%, de préférence entre 75 et 85%. La troisième température, T3, du flux de gaz froid est de préférence comprise entre 55 et 65°C, de préférence entre 58 et 62°C, et la troisième humidité relative, RH3 dudit flux de gaz refroidi est de préférence comprise entre 80 et 100%, de préférence entre 95 et 99%.

Pour une plus grande flexibilité dans la nature des matériaux à traiter, les premiers et second plateaux comprennent de préférence une structure rigide autoportante à haute perméabilité de type caillebotis, sur laquelle est posée une couche filtrante comprenant des ouvertures de taille et densité correspondant à la perméabilité désirée selon le type et taille des particules à traiter. L'entretien des plateaux est également facilité, avec la possibilité de changer la couche filtrante lorsqu'elle est endommagée ou colmatée.

Les premier et second moyens de répartition des particules sur les premier et second plateaux, respectivement, comprennent de préférence chacun au moins une vis d'Archimède s'étendant le long d'un rayon des premier et second plateaux, respectivement, ladite au moins une vis d'Archimède étant enfermée dans une enceinte munie d'une ou plusieurs ouvertures s'étendant le long dudit rayon des plateaux.

Le moyen de récupération du premier plateau comprend également de préférence au moins une vis d'Archimède s'étendant le long d'un rayon dudit plateau qui est enfermée dans une enceinte munie d'une ou plusieurs ouvertures s'étendant le long dudit rayon du premier plateau. Les ouvertures sont reliées à un racleur ou brosse apte à récolter et diriger les particules amenées par la rotation du plateau vers la vis d'Archimède. Dans une variante préférée, l'étuve comprend de plus un second moyen de récupération des particules réparties sur le second plateau après une rotation d'un angle donné de celui-ci, le dit second moyen de récupération étant situé en aval du, préférablement adjacent au second moyen de répartition, ledit moyen de récupération permettant de récupérer les particules sur le second plateau et de les transférer vers l'extérieur de l'enceinte. Le second moyen de récupération du second plateau comprend, par exemple, au moins une vis d'Archimède s'étendant le long d'un rayon dudit plateau qui est enfermée dans une enceinte munie d'une ou plusieurs ouvertures s'étendant le long dudit rayon du second plateau, lesdites ouvertures étant reliées à un racleur ou brosse apte à récolter et diriger les particules amenées par la rotation du plateau vers la vis d'Archimède.

L'axe vertical, Z, est de préférence centré sur le conduit de chauffage qui forme une enceinte centrale essentiellement cylindrique creuse dont la paroi s'étend au moins du premier plateau au second plateau. L'enceinte centrale peut ainsi contenir la soufflerie et la station de chauffage.

L'étuve peut comprendre un plancher statique situé en dessous du plateau inférieur situé le plus bas dudit axe vertical, Z. Le plancher comprend une ouverture d'évacuation des particules les plus fines qui se seraient déposées sur le plancher/ L'étuve peut de plus comprendre un racleur fixé de manière solidaire au plateau inférieur situé le plus bas et apte à suivre le mouvement de rotation de celui-ci pour pousser les particules déposées sur le plancher vers ladite ouverture d'évacuation.

Afin d'automatiser le traitement, le premier moyen de répartition desdites particules sur le premier plateau peut être relié en amont à une source desdites particules, de préférence un silo. Les particules comprennent de manière préférée des déchets ou sous-produits:
- de bois de scieries ou de bois de matériaux de construction; ou
- de papier ou cartons.

Lesdits déchets ou sous-produits peuvent être sous forme de poudre, de sciure, de granulés, de copeaux, de plaquette, de pellets, de tourteaux, et ou les particules ont de préférence une plus grande taille moyenne comprise entre 1 et 150 mm, de préférence entre 5 et 50 mm.

La présente invention concerne également un procédé de traitement de particules organiques d'origine végétale pour l'élimination d'organismes nuisibles présentant des risques phytosanitaires. Le procédé de la présente invention comprend l'utilisation d'une étuve telle que décrite ci-dessus pour effectuer les étapes suivantes,
(a) Former un flux de gaz chaud en soufflant à l'aide de la soufflerie de ladite étuve un gaz froid à travers la station de chauffage de l'étuve, et diriger le flux de gaz chaud ainsi formé à une première température, T1, et une première humidité relative, RH1, suivant un flux sensiblement parallèle à l'axe Z, passant d'abord à travers le premier plateau avant de passer directement après à travers le second plateau;
(b) Répartir les particules à traiter sur le premier plateau circulaire traversé en premier par le flux de gaz chaud et faire tourner le premier plateau autour de l'axe vertical, Z, à la première vitesse de rotation, v1, afin que les particules (20a) réparties sur le premier plateau atteignent une température de traitement, Tt, après une rotation d'un premier angle, θ, donné,
(c) Après rotation du premier plateau d'un angle, θ, donné, récupérer les particules ayant la température de traitement, Tt, dudit premier plateau et les transférer vers et répartir sur,
(d) le second plateau circulaire qui est traversé par un flux de gaz refroidi ayant une seconde température, T2 ≥ Tt, et une seconde humidité relative, RH2, après avoir traversé le premier plateau, et faire tourner le second plateau autour de l'axe vertical, Z, à la seconde vitesse de rotation, v2, afin de maintenir les particules à la température de traitement, Tt, pendant un temps, t1,
(e) Après rotation du second plateau d'un second angle, θ, donné, récupérer les particules ayant la température de traitement, Tt, dudit second plateau et les transférer hors de l'étuve, et
(f) Diriger le flux de gaz froid ayant une troisième température, T3 < T2, et une troisième humidité relative, RH3 > RH2, après avoir traversé le second plateau vers la soufflerie et répéter les étapes (a) à (f).

### BREVE DESCRIPTION DES FIGURES

Pour une meilleure compréhension de la nature de la présente invention, il est fait référence aux Figures suivantes.
**Figure 1** : illustre schématiquement deux variantes d'étuves selon la présente invention.
**Figure 2** : illustre (a) les flux de particules et de gaz à travers les plateaux de l'étuve de la présente invention et (b) une vue du dessus des plateaux avec indication des flux de particules.
**Figure 3** : illustre la température, Tp, des particules selon leur position angulaire sur les premier et second plateaux, respectivement.
**Figure 4** : illustre la température, Tg, et l'humidité relative, RH, du gaz à différentes positions dans l'étuve.
**Figure 5** : illustre différentes géométries de déflecteurs amonts et avals.
**Figure 6** : illustre différentes géométries de déflecteurs amonts et avals.

### DESCRIPTION DETAILLEE

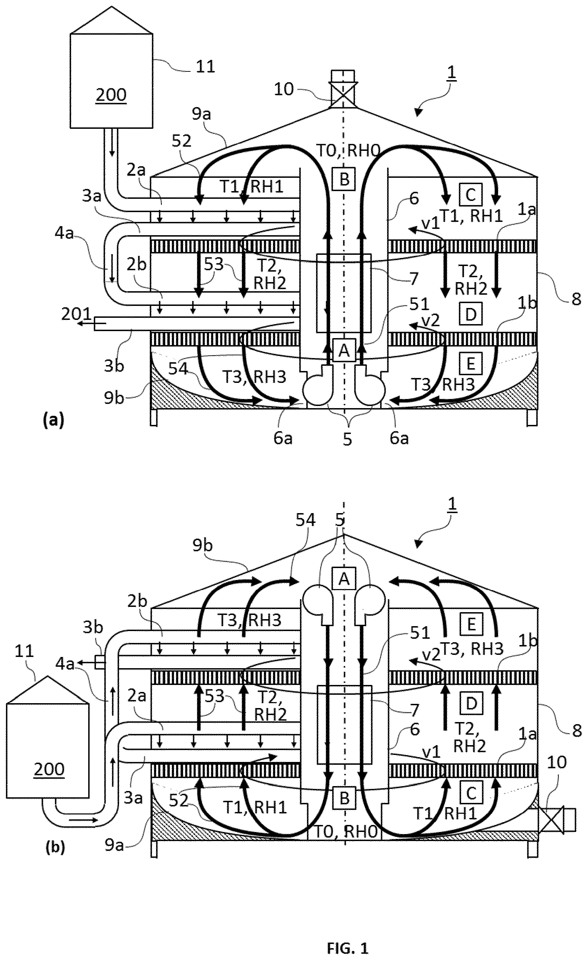

Telle qu'illustrée à la Figure 1, une étuve selon la présente invention est définie par enceinte (8) comprenant une paroi essentiellement cylindrique s'étendant le long d'un axe vertical, Z . Un premier plateau (1a) circulaire, dont la surface est perforée et perméable à l'air, à la vapeur d'eau et à l'eau, est monté sur la paroi de ladite enceinte (8) sensiblement normal à l'axe vertical, Z. Le premier plateau est agencé pour tourner à une première vitesse de rotation, v1, dans un premier sens autour de l'axe vertical, Z.

Un second plateau (1b) circulaire, dont la surface est perforée et perméable à l'air, à la vapeur d'eau et à l'eau, est monté à une certaine distance du premier plateau sur la paroi de ladite enceinte (8) sensiblement normal à l'axe vertical, Z. Le second plateau est agencé pour tourner à une seconde vitesse de rotation, v2, autour dudit axe vertical, Z. Les sens de rotation des premier et second plateaux peuvent être identiques ou inverses. De préférence le sens de rotation du second plateau est l'inverse de celui du premier plateau, La second vitesse de rotation, v2, est de préférence inférieure ou égale à la première vitesse de rotation, v1 : v1 = k v2 (ou v2 = 1/k v1), avec k ≥ 1. Une telle différence de vitesses de rotations permet, d'une part, aux particules (20a) se trouvant sur le premier plateau d'atteindre la température de traitement, Tt, après une rotation du premier plateau et, d'autre part, aux particules (20b) se trouvant sur le second plateau de maintenir la température de traitement, Tt, pendant le temps de traitement, t1, au cours d'une rotation du second plateau.

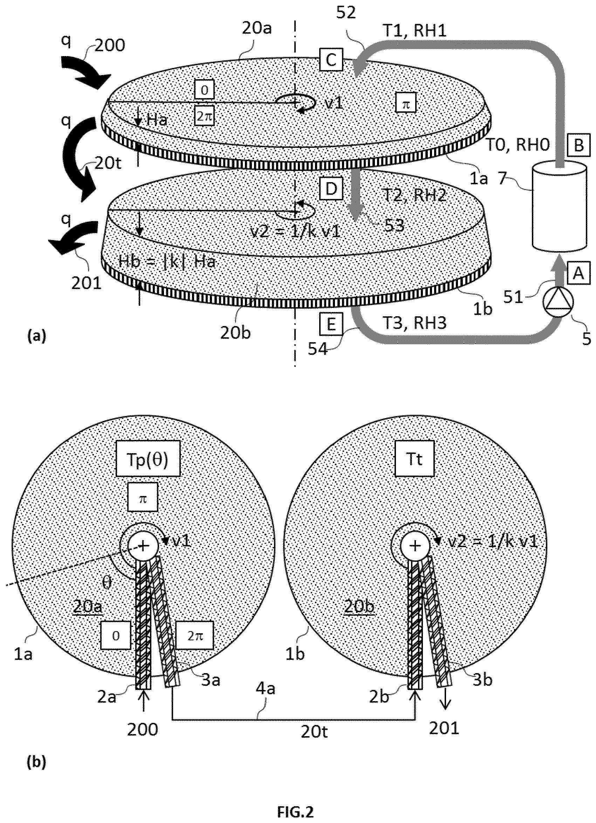

Un premier moyen de répartition (2a) desdites particules est agencé au-dessus du premier plateau, s'étendant de préférence le long d'un rayon dudit premier plateau. Le premier moyen de répartition permet de répartir lesdites particules (200) à traiter, avant étuvage le long d'un rayon du premier plateau (1a). Un premier moyen de récupération (3a) est agencé en aval du premier moyen de répartition (2a). il permet de récupérer les particules réparties sur le premier plateau (1a) après une rotation d'un angle donné de celui-ci. L'angle donné est le plus proche possible de 360° ; il est par exemple de 340 à 359°. Dans ce cas le dit premier moyen de récupération s'étend de préférence le long d'un rayon du premier plateau et est adjacent au premier moyen de répartition (2a). Comme indiqué à la Figure 2(b), l'angle de rotation, θ, d'un plateau est mesuré à partir du moyen de répartition correspondant.

Un moyen de transfert (4a) permettant de transférer vers un second moyen de répartition (2b) des particules (20t) récoltées du premier plateau (1a) par le premier moyen de récupération (3a). Le second moyen de répartition (2b) est destiné à répartir lesdites particules le long d'un rayon du second plateau (1b). Les termes « amont » et « aval » sont définis ici par rapport au sens de déplacement des particules ou du gaz, selon les cas.

Le second moyen de répartition (2b) s'étend de préférence le long d'un rayon dudit second plateau. Dans une variante préférée de l'invention, le second plateau (1b) comprend également un moyen de récupération (3b) des particules déposées sur le second plateau après une rotation d'un angle donné de celui-ci. Comme pour le moyen de récupération (3a) du premier plateau discuté supra, le second moyen de récupération est situé en aval du second moyen de répartition (2b). Afin de maximiser l'angle de rotation, le second moyen de récupération s'étend de préférence le long d'un rayon du second plateau et est adjacent au second moyen de répartition.

L'étuve de la présente invention comprend de plus un moyen de soufflage de gaz formant un cycle gazeux fermé. Tel qu'illustré aux Figures 1&2(a), le moyen de soufflage de gaz comprend : une soufflerie (5), comprenant par exemple un ou plusieurs ventilateurs, pour donner une vitesse à un flux de gaz (51) et le diriger vers une station de chauffage (7) pour former un flux de gaz chaud (52) ayant une température initiale, T0, et une humidité relative initiale, RH0. La station de chauffage peut être située dans un conduit de chauffage (6) centré sur l'axe vertical Z formant une enceinte centrale essentiellement cylindrique creuse dont la paroi s'étend au moins du premier plateau (1a) au second plateau (1b), tel qu'illustré à la Figure 1. Alternativement, la station de chauffage peut se trouver hors de l'enceinte et profiter d'une source de chaleur disponible à l'extérieur. Après avoir accumulé de l'énergie calorifique dans la station de chauffage, le flux de gaz chaud se dirige vers un déflecteur amont, qui dévie le flux de gaz chaud vers le premier plateau (1a), suivant un flux sensiblement parallèle à l'axe Z. Avant d'atteindre le premier plateau, le gaz a une première température, T1, égale ou légèrement inférieure à T0, et une première humidité relative, RH1, égale ou légèrement supérieure à RH0, si T1 < T0.

Le gaz passe alors d'abord à travers les particules (20a) réparties sur la surface perforée du premier plateau (1a), où il perd de l'énergie calorifique et cinétique. Le gaz ressort du premier plateau formant un flux de gaz refroidi (53) ayant une seconde température, T2, inférieure à T1, et une seconde humidité relative, RH2, supérieure à RH1. Le flux de gaz refroidi (53) continue sa course pour ensuite passer directement après à travers les particules (20b) réparties sur la surface perforée du second plateau (1b), où il perd encore de l'énergie calorifique et d'où en ressort un flux de gaz froid (54) ayant une troisième température, T3, inférieure à T2, et une troisième humidité relative, RH3, supérieure à RH2. Les termes « gaz chaud », « gaz refroidi » et « gaz froid » sont des termes relatifs les uns par rapport aux autres, tels que T1 > T2 > T3, où T1, T2, et T3 sont les températures des gaz « chaud », « refroidi » et « froid », respectivement.

Un déflecteur aval permet de dévier le flux de gaz refroidi vers la soufflerie et recommencer le cycle gazeux. Contrairement à un sécheur, l'objectif de l'étuve de la présente invention n'est pas d'évacuer de l'humidité hors des particules mais de porter les particules à une température, Tt, et de les y maintenir pendant un temps, t1. Pour cette raison, même si le gaz s'est chargé d'humidité pendant un premier cycle, il n'est pas nécessaire de l'évacuer ou de le sécher pour le cycle suivant. Au contraire, l'eau étant un bon conducteur thermique, un certain taux d'humidité contribue à accélérer le transfert thermique du gaz vers les particules. Contrairement à un sécheur, une étuve selon la présente invention fonctionne donc de préférence avec un cycle gazeux fermé. Une valve (10) est cependant avantageusement agencée afin de permettre l'évacuation d'une partie au moins du gaz d'un cycle et de le remplacer par du gaz frais, si cela devait se révéler nécessaire.

Les premiers et second plateaux (1a, 1b) sont de préférence composés d'une structure rigide autoportante à haute perméabilité de type caillebotis. Une couche filtrante comprenant des ouvertures de taille et densité correspondant à la perméabilité désirée selon le type et taille des particules à traiter peut alors être posée directement sur la structure rigide. Ainsi, une même étuve peut être utilisée pour traiter des particules de tailles très différentes, simplement en changeant la couche filtrante. Celle-ci peut être une tôle perforée, un tamis, une grille ou une toile tissée de fibres végétales (par exemple, chanvre, coton), synthétique (par exemple, polyéthylène, polypropylène, polyester), ou métallique (par exemple, acier). Alternativement, la couche filtrante peut être formée d'une bâche perforée de trous de taille et densité adéquates aux particules à traiter.

Des particules d'origine végétales selon la présente invention comprennent par exemple des particules de bois, d'écorces, des cônes. Elles peuvent se présenter sous forme de laine de bois, de pellets, de copeaux, de sciures, de fibres, de poudres, de plaquettes, de tourteaux, etc. Les particules peuvent avoir une plus grande taille moyenne comprise entre 1 et 150 mm, de préférence entre 5 et 50 mm, où la « plus grande taille » est la distance séparant les deux points les plus éloignés l'un de l'autre d'une particule. Les particules peuvent par exemple être des déchets ou sous-produits de bois de scieries ou de bois de matériaux de construction, ou même de papier ou de cartons. Les particules peuvent être avantageusement stockées dans un silo (11) ou tout autre contenant de stockage, relié directement au premier moyen de répartition (2a) permettant ainsi de répartir les particules directement depuis leur endroit de stockage sur le premier plateau.

Le premier moyen de répartition (2a) des particules à traiter sur le premier plateaux (1a) a pour but de répartir les particules à chauffer de manière homogène le long d'un rayon du premier plateau. De manière générale, le premier moyen de répartition (2a) comprend donc:
- une structure s'étendant de la périphérie extérieure à la périphérie intérieure du premier plateau, suivant de préférence, mais pas nécessairement, un rayon de celui-ci,
- des moyens de transport des particules de la périphérie extérieure à la périphérie intérieure du premier plateau, et enfin
- des moyens de déposition desdites particules depuis le moyen de transport vers le premier plateau.

Plusieurs solutions sont possibles. Par exemple, le transport des particules de la périphérie extérieure vers le centre du premier plateau peut être assuré par une bande transporteuse, soit perforée, soit inclinée transversalement de sorte à permettre aux particules de saupoudrer le plateau situé en-dessous. Pour assister au saupoudrage, la bande peut être vibrée. Dans une variante alternative et préférée illustrée à la Figure 2(b), le premier moyen de répartition (2a) comprend au moins une vis d'Archimède s'étendant le long d'un rayon du premier plateau (1a), afin de transporter les particules de la périphérie extérieure vers la périphérie intérieure du plateau correspondant. Ladite au moins une vis d'Archimède est enfermée dans une enceinte munie d'une ou plusieurs ouvertures s'étendant vers le bas et le long dudit rayon du premier plateaux (1a) afin de permettre le saupoudrage des particules sur ledit plateau.

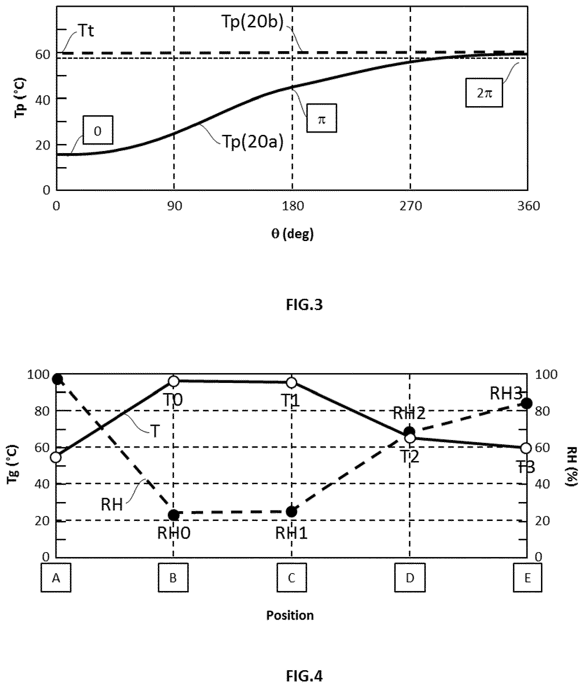

Les particules s'accumulent donc sur un rayon du premier plateau à un angle de rotation θ de 0° (cf. 0 aux Figures 2&3). Comme le premier plateau tourne à une vitesse, v1, autour de l'axe Z, une couche homogène de particules (20a) de hauteur Ha, recouvre la surface du premier plateau. Comme illustré à la Figure 3, pendant la rotation du premier plateau, les particules sont chauffées par le flux de gaz chaud (52) et leur température, Tp, augmente avec l'angle de rotation, θ, du premier plateau (Tp = Tp(θ)). La vitesse de rotation, v1, du premier plateau est définie afin d'assurer que les particules (20a) atteignent la température de traitement, Tt, après que le premier plateau ait tourné d'un angle donné, inférieur ou égal à, et aussi proche de 360° que possible (cf. 2π aux Figures 2&3). La vitesse de rotation, v1, dépend donc du type et des propriétés du lit de particules et des caractéristiques du flux de gaz chaud (52), incluant sa température, T1, son humidité relative, RH1, et son débit. La température de traitement, Tt, est de préférence comprise entre 55 et 80°C, de préférence Tt ≥ 60°C.

A ce point, les particules (20a) sont recueillies à une température ≥ Tt par le moyen de récupération (3a) pour être transférées vers le second plateau (1b). Comme illustré à la Figure 2(b), le moyen de récupération (3a) du premier plateau (1a), comprend de préférence au moins une vis d'Archimède s'étendant le long d'un rayon dudit plateaux qui est enfermée dans une enceinte munie d'une ou plusieurs ouvertures s'étendant le long dudit rayon du plateau correspondant. Les ouvertures sont reliées à un racleur ou brosse apte à récolter et diriger les particules amenées par la rotation du plateau vers la vis d'Archimède. Le type de moyen de transfert (4a) des particules du premier plateau (1a) vers le second plateau (1b) dépend de la configuration de l'étuve. Si le premier plateau (1a) est le plateau supérieur, le moyen de transfert peut être un simple tube reliant le moyen de récupération (3a) du premier plateau au moyen de répartition (2b) du second plateau, dans lequel les particules tombent par gravité. Si au contraire, le premier plateau est le plateau inférieur, il est préférable que le moyen de transfert (4a) comprenne une vis d'Archimède permettant de monter les particules du premier plateau inférieur vers le second plateau supérieur.

Les particules (20t) sont ainsi transférées vers un second moyen de répartition (2b) qui répartit les particules de manière homogène sur la surface du second plateau (1b). Le second moyen de répartition peut être du même type que le premier moyen de répartition discuté plus haut. Généralement, mais pas nécessairement, les premier et second moyens de répartition sont identiques. Comme illustré à la Figure 2(b), le second moyen de répartition (2b) comprend de préférence au moins une vis d'Archimède s'étendant le long d'un rayon du second plateau (1b). L'au moins une vis d'Archimède est enfermée dans une enceinte munie d'une ou plusieurs ouvertures s'étendant le long dudit rayon du plateau (1b). Les particules s'accumulent donc sur un rayon du second plateau à un angle de rotation θ de 0°.

Comme le second plateau tourne à une vitesse, v2, autour de l'axe Z, une couche homogène de particules (20b) de hauteur Hb, recouvre la surface du second plateau. La vitesse de rotation, v2, du second plateau est généralement différente de la vitesse de rotation, v1, du premier plateau. En effet, si la vitesse de rotation, v1, est optimisée pour que les particules (20a) atteignent la température de traitement, Tt, après un tour du premier plateau (c'est-à-dire après une rotation des particules partant du premier moyen de répartition (2a) jusqu'au moyen de récupération (3a)), la vitesse de rotation, v2, du second plateau dépend du temps, t1, que les particules doivent rester à la température, Tt. Par exemple, si on prend un traitement thermique tel que défini dans la norme NIMP 15 imposant un traitement à une température de traitement, Tt, d'au moins 56°C pendant un temps, t1, de 30 min, la vitesse de rotation, v2, du second plateau sera environ égale à v2 ≃ 360 deg / 30 min = 12 deg / min. Comme illustré à la Figure 3, pendant la rotation du second plateau, les particules sont maintenues par le flux de gaz refroidi (53) à leur température de traitement, Tt, pendant toute la rotation du second plateau.

La vitesse de rotation, v1, du premier plateau peut être exprimée en fonction de la vitesse de rotation, v2, du second plateau comme : v1 = k v2. Si les premier et second plateaux tournent dans des sens inverses, k sera négatif. Dans la plupart des cas, le temps d'exposition au flux de gaz chaud (52) des particules (20a) posées sur le premier plateau nécessaire à les chauffer à une température de traitement, Tt, est inférieur au temps, t1, pendant lequel les particules (20b) doivent demeurer à la température de traitement, Tt. Si la vitesse de rotation, v1, du premier plateau est supérieure ou égale à la vitesse, v2, du second plateau, la valeur absolue de k est alors supérieure ou égale à 1 (|v1| ≥ |v2| ⇔ |k| ≥ 1). Par exemple, si v2 est compris entre 0.5 et 1.2 tours par heure, la valeur absolue de k peut être comprise entre 1 et 5, de préférence entre 2 et 4, et encore de préférence, |k| = 3 ± 0.5.

Par le principe de conservation de masse, les épaisseurs, Ha et Hb, des couches de particules (20a, 20b) se trouvant sur les premier et second plateaux (1a, 1b) dépendent directement des débits, q, de répartition des particules sur les plateaux respectifs, et des vitesses de rotation. Les particules à traiter (200) sont réparties sur le premier plateau à un débit, q [kg / s]. Le premier plateau tourne à une vitesse, v1, pendant un tour avant de récupérer les particules (20a) et de les transférer vers le second plateau. Là, elles sont réparties sur le second plateau au même débit, q, que pour le premier plateau (cf. Figure 2(a)). Le second plateau tourne à une vitesse, v2, pendant un tour avant de récupérer les particules (20b) et évacuer les particules (201) au même débit, q, que précédemment défini. Comme les débits, q, des particules (200), (20t) et (201) sont égaux, les hauteurs Ha et Hb peuvent être exprimées comme, Hb = |k| Ha.

Le gaz chaud, par exemple de l'air chaud ou tout autre gaz issu par exemple d'un procédé de combustion, suit un parcours dans le même sens que celui des particules, soit passant d'abord par le premier plateau pour chauffer les particules (20a) à la température de traitement, Tt, puis par le second plateau pour les maintenir à ladite température de traitement, Tt, pendant un temps, t1. Sur le graphe de la Figure 4, le flux de gaz (51) sortant d'une soufflerie (5) a une température, Tg, et une humidité relative, RH, données (voir position A sur les Figures 1, 2 et 4). A ce stade, le gaz, par exemple de l'air, est à sa température la plus basse. Par exemple la température du flux de gaz (51) est de l'ordre de 55°C. Le point de rosée de l'air dépendant de la température, l'humidité relative, RH, du flux de gaz (51) est la plus élevée. Par exemple, RH = 100%. Le flux de gaz passe à travers une station de chauffage (7) afin d'en augmenter la température à une valeur, T0, ce qui a pour effet de baisser la valeur de l'humidité relative, RH0 (voir position B sur les Figures 1, 2 et 4). Par exemple, la température, T0, du flux de gaz chaud peut être comprise entre 75 et 120°C, de préférence entre 85 et 100°C, encore de préférence entre 90 et 95°C. L'humidité relative, RH0 dudit flux de gaz chaud peut être comprise entre 15 et 60%, de préférence entre 30 et 40%.

A la sortie de la station de chauffage, le flux de gaz chauffé est dévié pour s'orienter parallèle à l'axe Z, pour se diriger vers le premier plateau. La première température, T1, et la première humidité relative, RH1, du flux de gaz (52) sont sensiblement identique à T0 et RH0, différant seulement du fait de la baisse de température, T1 par rapport à T0, par des effets de pertes (mauvaise isolation, etc.) (voir position C sur les Figures 1, 2 et 4). Comme pour T0, la première température, T1, du flux de gaz chaud (52) peut être comprise entre 75 et 120°C, de préférence entre 85 et 100°C, encore de préférence entre 90 et 95°C, et la première humidité relative, RH1 dudit flux de gaz chaud peut être comprise entre 15 et 60%, de préférence de préférence RH1 ≥ 20%,. En traversant le lit de particules (20a) et le premier plateau, le flux de gaz (52) transfère une partie de son énergie et les particules (20a) sont chauffées en fonction du temps d'exposition au flux de gaz, et donc en fonction de la position, θ, sur premier plateau (Tp = Tp(θ), voir Figure 3, #Tp(20a)).

Le flux de gaz refroidi (53) après avoir traversé le premier plateau est donc refroidi à une seconde température, T2 < T1. Il perd donc de l'énergie calorifique, mais aussi une partie de son énergie cinétique en traversant le lit de particules (20a) et la surface perforée du premier plateau (1a). L'humidité relative augmente donc à une seconde valeur, RH2 > RH1 (voir position D sur les Figures 1, 2 et 4). Par exemple, la seconde température, T2, du flux de gaz refroidi est comprise entre 60 et 80°C, de préférence entre 65 et 70°C, et la seconde humidité relative, RH2 dudit flux de gaz refroidi est comprise entre 60 et 90%, de préférence entre 75 et 85%,

Le flux de gaz froid (54) après avoir traversé le second plateau est donc refroidi à une troisième température, T3 < T2 < T1. Il perd donc de l'énergie calorifique, mais aussi une partie de son énergie cinétique en traversant le lit de particules (20b) et la surface perforée du second plateau (1b). L'humidité relative augmente donc à une troisième valeur, RH3 > RH2 > RH1 (voir position E sur les Figures 1, 2 et 4). Par exemple, la troisième température, T3, du flux de gaz froid est comprise entre 55 et 65°C, de préférence entre 58 et 62°C, et la troisième humidité relative, RH3 dudit flux de gaz froid est comprise entre 80 et 100%, de préférence entre 95 et 99%. Le gaz utilisé pour traiter les particules peut être tout type de gaz ne présentant pas de danger d'explosion ou de toxicité ou pollution. Le gaz peut préférablement être de l'air.

Dans une première variante de l'invention, illustrée aux Figures 1(a), 2(a) et 5, le premier plateau (1a) est situé en dessus du second plateau (1b). Le gaz chaud circule alors du haut vers le bas. Cette variante a l'avantage de souffler les particules contre les surfaces des plateaux, permettant de diminuer les poussières en suspension. Cependant, les lits de particules déposés sur les premier et second plateaux sont ainsi densifiés en en diminuant la perméabilité aux gaz et rendant plus difficile le chauffage individuel des particules. Cette variante est donc préférée pour traiter des particules très légères ou fines ou, au contraire, des particules assez grosses, formant un lit de haute perméabilité aux gaz, même si comprimés.

Dans une seconde variante de la présente invention, illustrée aux Figures 1(b)&6, le premier plateau (1a) est situé au-dessous du second plateau (1b). Le gaz chaud circule alors du bas vers le haut. Si les particules sont très légères, un nuage en suspension peut se former ce qui est à éviter. Par contre, si les particules ont un poids adéquat, une telle variante est avantageuse en ce qu'un lit fluidisé peut ainsi être formé, ce qui permet au gaz chaud d'atteindre pratiquement chaque particule individuellement, augmentant ainsi l'efficacité du transfert de chaleur vers les particules. Le choix de l'une ou de l'autre variante concernant la position relative des premier et second plateaux dépend donc de la nature des particules à traiter et des flux de gaz utilisés.

Les déflecteurs amont et aval (9a, 9b) ne doivent pas avoir une géométrie particulière à condition qu'ils permettent de changer l'orientation du flux de gaz. Par exemple, dans le cas d'une enceinte (8) cylindrique, un toit par exemple plat ou conique et un plancher horizontal peuvent former les déflecteurs amont et aval. En effet, quelle que soit l'orientation du flux de gaz chaud (52) entrant dans l'enceinte en amont du premier plateau (1a), il sera nécessairement dévié vers la surface perforée du premier plateau par le toit ou le plancher, selon où se trouve le premier plateau, faisant ainsi office de déflecteur amont (9a). De manière similaire le flux de gaz froid (54) en aval du second plateau est nécessairement dévié vers la soufflerie par le plancher ou toit, selon la position du deuxième plateau, faisant ainsi office de déflecteur aval (9b).

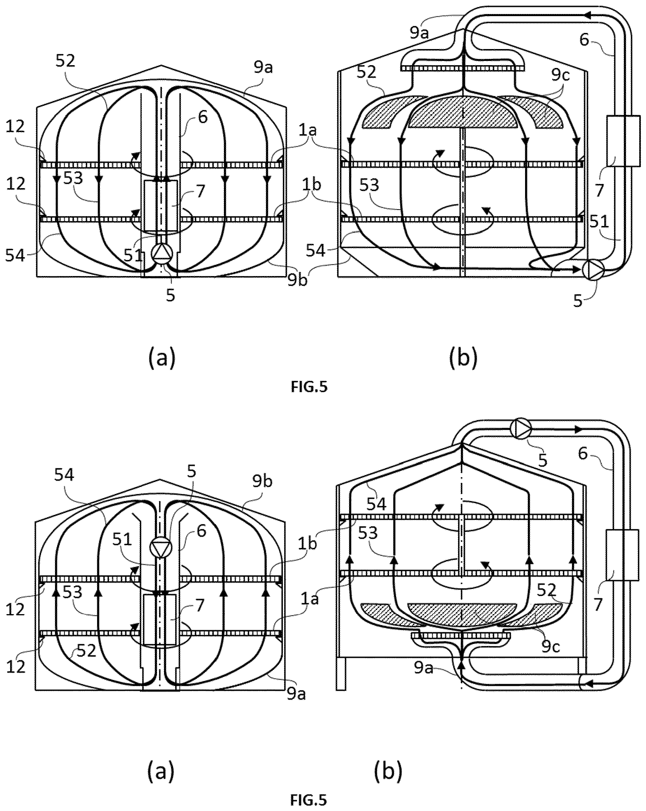

Les déflecteurs amont et aval (9a, 9b) peuvent de préférence avoir une géométrie profilée permettant de dévier les flux de gaz chaud (52) et froid (54) en réduisant les turbulences et lissant les flux de manière laminaire ou presque. Ainsi, comme illustré sur les Figures 5&6, le déflecteur amont (9a) permet d'orienter le gaz chaud (52) sensiblement normal à la surface du premier plateau. Tel qu'illustré sur les Figures 5(a)&6(a), si le conduit de chauffage (6) forme une enceinte centrée sur l'axe vertical, Z, essentiellement cylindrique creuse, le déflecteur amont (9a) peut être formé par une voûte dans le plafond ou sur le plancher de l'enceinte, selon que le premier plateau (1a) se trouve au-dessus ou en-dessous du second plateau, respectivement. La voûte peut être courbe comme illustré sur les Figures 5(a)&6(a) ou conique. Comme illustré sur les Figures 5(b)&6(b), dans le cas d'un conduit de chauffage (6) situé à l'extérieur de l'enceinte de l'étuve, ledit conduit de chauffage comprend un coude (9a) permettant de dévier le flux de gaz dans la direction normale au premier plateau. Le conduit de chauffage peut être muni à son extrémité avale d'un pommeau de distribution muni d'une grille. Un distributeur amont (9c) peut être situé en aval de l'extrémité avale du conduit de chauffage pour assurer que le gaz chaud (52) soit distribué sur l'ensemble de la surface du premier plateau.

Afin d'éviter que le gaz chaud ne court-circuite les particules posées sur les plateaux et ne passe par la périphérie des plateaux, entre la circonférence d'un plateau et l'enceinte de l'étuve, des moyens (12) pour étanchéifier suffisamment la circonférence des plateaux sont prévus. Par exemple, une jupe peut s'étendre depuis l'enceinte de l'étuve et recouvrir une partie de la surface amont de chaque plateau sur leur circonférence (cf. Figures 5&6). Les plateaux peuvent également s'emboîter dans une rainure pourvue sur la surface intérieure de l'enceinte de l'étuve. Tout autre moyen d'étanchéité dynamique connu par l'homme du métier peut être utilisé sans modifier la présente invention.

Les déflecteurs avals permettent de dévier le gaz froid (54) après avoir traversé le second plateau (1b) vers le ou les ventilateurs formant la soufflerie (5) ou compresseur qui redonne au flux de gaz froid de l'énergie cinétique avant de l'envoyer vers la station de chauffage (7) dans le conduit de chauffage (6). Dans le cas d'un conduit de chauffage (6) centré sur l'axe vertical Z, comme illustré sur les Figures 5(a)&6(a), les déflecteurs avals (9b) permettent de rediriger le flux de gaz froid (54) vers des ouvertures distribuées sur la périphérie du conduit de chauffage, vers la soufflerie permettant de redonner de l'énergie cinétique au flux de gaz froid et de le diriger vers la station de chauffage pour lui redonner de l'énergie calorifique. Dans le cas d'un conduit de chauffage (6) extérieur tel qu'illustré sur les Figures 5(b)&6(b), les déflecteurs avals (9b) permettent de diriger le flux d'air froid (54) vers un orifice situé dans la paroi de l'enceinte ou dans le toit de l'étuve donnant accès au conduit de chauffage. Les déflecteurs avals (9b) peuvent être formés par une surface à double courbure, ou conique, ou être formée par des surfaces planes distribuées sur la circonférence de l'enceinte de l'étuve en aval du second plateau.

Les Figures illustrent des étuves comprenant deux plateaux. Cependant, pour réduire l'espace au sol occupé par l'équipement, il est tout à fait possible de monter :
- au moins un troisième plateau circulaire monté sensiblement horizontalement à une certaine distance, et séparé du premier plateau (1a) par, le second plateau (1b), en rotation autour dudit axe vertical, Z, la surface dudit plateau étant perforée et perméable à l'air, à la vapeur d'eau et à l'eau, et
- Un moyen de transfert des particules récoltées du second plateau (1b) par le moyen de récupération (3b) vers un troisième moyen de répartition apte à répartir lesdites particules le long d'un rayon du troisième plateau.

En vue de la distribution de la granulométrie des particules d'un même type, il est difficile d'éviter que la fraction la plus fine des particules ne passe à travers les perforations des plateaux et ne tombe sur le ou les plateaux inférieurs, puis sur le plancher de l'enceinte de l'étuve enfermant les plateaux. Afin d'éviter une trop grande accumulation de particules sur le plancher et aussi pour les récupérer, il est avantageux de munir le plancher d'une ouverture d'évacuation des particules les plus fines qui se seraient déposées sur le plancher. De plus, un racleur ou brosse fixé de manière solidaire au plateau inférieur et apte à suivre le mouvement de rotation de celui-ci sert à pousser les particules déposées sur le plancher vers ladite ouverture d'évacuation. Comme le racleur ou brosse est fixé au plateau inférieur, il n'est pas nécessaire de le motoriser individuellement.

La présente invention concerne également un procédé de traitement de particules organiques d'origine végétale pour l'élimination d'organismes nuisibles présentant des risques phytosanitaires. Le procédé de la présente invention utilise une étuve telle que discutée supra et comprend les étapes suivantes,
(a) Former un flux de gaz chaud (52) en soufflant à l'aide de la soufflerie (5) de ladite étuve (1) un gaz froid (51) à travers la station de chauffage (7) de l'étuve, et diriger le flux de gaz chaud (52) ainsi formé à une première température, T1, et une première humidité relative, RH1, suivant un flux sensiblement parallèle à l'axe Z, passant d'abord à travers le premier plateau (1a) avant de passer directement après à travers le second plateau (1b) ;
(b) Répartir les particules (200) à traiter sur le premier plateau (1a) circulaire traversé en premier par le flux de gaz chaud (52) et faire tourner le premier plateau autour de l'axe vertical, Z, à la première vitesse de rotation, v1, afin que les particules (20a) réparties sur le premier plateau atteignent une température de traitement, Tt, après une rotation d'un premier angle, θ, donné,
(c) Après rotation du premier plateau d'un angle, θ, donné, récupérer les particules ayant la température de traitement, Tt, dudit premier plateau et les transférer vers et répartir sur,
(d) le second plateau (1b) circulaire qui est traversé par un flux de gaz refroidi (53) ayant une seconde température, T2 ≥ Tt, et une seconde humidité relative, RH2, après avoir traversé le premier plateau, et faire tourner le second plateau autour de l'axe vertical, Z, à la seconde vitesse de rotation, v2, afin de maintenir les particules à la température de traitement, Tt, pendant un temps, t1,
(e) Après rotation du second plateau d'un second angle, θ, donné, récupérer les particules (20b) ayant la température de traitement, Tt, dudit second plateau et les transférer hors de l'étuve, et
(f) Diriger le flux de gaz froid (54) ayant une troisième température, T3 < T2, et une troisième humidité relative, RH3 > RH2, après avoir traversé le second plateau vers la soufflerie et répéter les étapes (a) à (f).

L'étuve de la présente invention permet de traiter en continu selon les normes internationales des particules organiques d'origine végétale de tailles et de natures très différentes pour l'élimination d'organismes nuisibles présentant des risques phytosanitaires. L'énergie requise pour le traitement est optimisée en séparant les étapes de chauffage à une température, Tt, de traitement sur un premier plateau et de maintien des particules à cette température, Tt, pendant un temps, t1, de traitement sur un second plateau. L'étuve est simple et économique à fabriquer, d'entretien facile et assure une reproductibilité et constance du traitement appliqué aux particules.

| **REF** | **DEFINITION** |
|---|---|
| **1** | étuve |
| **1a** | premier plateau |
| **1b** | second plateau |
| **2a** | premier moyen de répartition |
| **2b** | second moyen de répartition |
| **3a** | premier moyen de récupération |
| **3b** | second moyen de récupération |
| **4a** | premier moyen de transfert |
| **5** | soufflerie |
| **6** | conduit de chauffage |
| **6a** | fenêtre d'entrée dans le conduit de chauffage |
| **7** | station de chauffage |
| **8** | enceinte de l'étuve |
| **9a** | déflecteur amont |
| **9b** | déflecteur aval |
| **9c** | distributeur amont |
| **10** | cheminée munie d'une valve |
| **11** | silo |
| **12** | moyens pour étanchéifier la circonférence des plateaux |
| **20a** | particules se trouvant sur le premier plateau |
| **20b** | particules se trouvant sur le second plateau |
| **20t** | particules en transfert du premier plateau vers le second plateau |
| **51** | flux de gaz sortant de la soufflerie |
| **52** | flux de gaz chaud sortant de la station de chauffage |
| **53** | flux de gaz chaud ayant traversé le premier plateau, mais pas le second |
| **54** | flux de gaz refroidi ayant traversé le second plateau |
| **200** | particules à traiter |
| **201** | particules après le traitement |
| **q** | débit de particules (kg / s) |
| **Ha** | hauteur du lit de particules sur le premier plateau (1a) |
| **Hb** | hauteur du lit de particules sur le second plateau (1b) |
| **k** | facteur de proportionnalité entre v1 et v2, v1 = k v2, k ≥ 1 |
| **RH0** | humidité relative initiale du gaz (à la sortie de la station de chauffage |
| **RH1, 2, 3** | première, seconde, et troisième humidité relative du gaz |
| **t1** | temps de traitement à la température Tt ou supérieure |
| **T0** | température initiale du gaz (à la sortie de la station de chauffage |
| **T1, 2, 3** | première, seconde, et troisième température du gaz |
| **Tt** | température de traitement des particules |
| **v1** | vitesse de rotation du premier plateau |
| **v2** | vitesse de rotation du second plateau |
| **θ** | angle de rotation d'un plateau à partir du moyen de répartition correspondant |

## Revendications

1. Etuve (1) pour l'élimination d'organismes nuisibles présentant des risques phytosanitaires présents dans des matériaux d'origine végétale sous forme de particules, ladite étuve comprenant,
(a) Une enceinte (8) comprenant une paroi essentiellement cylindrique s'étendant le long d'un axe vertical, Z,
(b) Un premier plateau (1a) circulaire monté sur la paroi de ladite enceinte (8) sensiblement normal à l'axe vertical, Z, et agencé pour tourner à une première vitesse de rotation, v1, dans un premier sens autour de l'axe vertical, Z, la surface dudit plateau étant perforée, et perméable à l'air, à la vapeur d'eau et à l'eau,
(c) Un second plateau (1b) circulaire monté à une certaine distance du premier plateau sur la paroi de ladite enceinte (8) sensiblement normal à l'axe vertical, Z, et agencé pour tourner à une seconde vitesse de rotation, v2, autour dudit axe vertical, Z, de préférence dans le sens inverse de rotation du premier plateau, la surface dudit plateau étant perforée et perméable à l'air, à la vapeur d'eau et à l'eau,
(d) Un premier moyen de répartition (2a) desdites particules apte à répartir lesdites particules avant étuvage le long d'un rayon du premier plateau (1a),
(e) Un premier moyen de récupération (3a) des particules (20a) réparties sur le premier plateau (1a) après une rotation d'un angle donné de celui-ci, le dit premier moyen de récupération étant situé en aval du, et préférablement adjacent au premier moyen de répartition (2a),
(f) Un moyen de transfert (4a) des particules récoltées du premier plateau (1a) par le premier moyen de récupération (3a) vers un second moyen de répartition (2b) apte à répartir lesdites particules (20t) le long d'un rayon du second plateau (1b), et
(g) Un moyen de soufflage de gaz formant un cycle gazeux fermé, comprenant :
• une soufflerie (5) pour donner une vitesse à un flux de gaz (51) et le diriger vers,
• une station de chauffage (7) pour former un flux de gaz chaud (52) ayant une température initiale, T0, et une humidité relative initiale, RH0, et ensuite le diriger vers,
• un déflecteur amont, déviant le flux de gaz chaud suivant un flux sensiblement parallèle à l'axe Z, ayant une première température, T1, et une première humidité relative, RH1, passant d'abord à travers la surface perforée du premier plateau (1a), où il perd de l'énergie calorifique et d'où en ressort un flux de gaz refroidi (53) ayant une seconde température, T2, et une seconde humidité relative, RH2, pour ensuite passer directement après à travers la surface perforée du second plateau (1b), où il perd encore de l'énergie calorifique et d'où en ressort un flux de gaz froid (54) ayant une troisième température, T3, et une troisième humidité relative, RH3, pour ensuite atteindre,
• un déflecteur aval déviant le flux de gaz froid (54) vers la soufflerie et recommencer le cycle gazeux.

2. Etuve (1) selon la revendication 1, dans lequel le premier plateau (1a) est situé en dessus du second plateau (1b) et où le gaz chaud circule du haut vers le bas et est préférablement de l'air chaud.

3. Etuve (1) selon la revendication 1, dans lequel le premier plateau (1a) est situé au-dessous du second plateau (1b) et où le gaz chaud circule du bas vers le haut et est préférablement de l'air chaud.

4. Etuve (1) selon l'une quelconque des revendications précédentes, comprenant un contrôleur configuré pour contrôler que la première vitesse de rotation, v1, du premier plateau soit supérieure à la seconde vitesse de rotation, v2, du second plateau, avec v2 = 1/k · v1, dans lequel, |k| ≥ 1, et la valeur absolue de k est de préférence compris entre 1 et 5, de préférence entre 2 et 4, encore de préférence, |k| = 3, et dans lequel v2 est de préférence compris entre 0.5 et 1.2 tours par heure.

5. Etuve (1) selon l'une quelconque des revendications précédentes, comprenant un contrôleur configuré pour contrôler que la première température, T1, du flux de gaz chaud (52) soit comprise entre 75 et 120°C, de préférence entre 85 et 100°C, encore de préférence entre 90 et 95°C, et la première humidité relative, RH1 dudit flux de gaz chaud soit comprise entre 15 et 60%, de préférence RH1 ≥ 20%.

6. Etuve (1) selon l'une quelconque des revendications précédentes, comprenant un contrôleur configuré pour contrôler que,
• la seconde température, T2, du flux de gaz refroidi (53) soit comprise entre 60 et 80°C, de préférence entre 65 et 70°C, et la seconde humidité relative, RH2 dudit flux de gaz refroidi soit comprise entre 60 et 90%, de préférence entre 75 et 85%, et que,
• la troisième température, T3, du flux de gaz froid (54) soit comprise entre 55 et 65°C, de préférence entre 58 et 62°C, et la troisième humidité relative, RH3 dudit flux de gaz refroidi est comprise entre 80 et 100%, de préférence entre 95 et 99%

7. Etuve (1) selon l'une quelconque des revendications précédentes, dans laquelle les premiers et second plateaux (1a, 1b) comprennent une structure rigide autoportante à haute perméabilité de type caillebotis, sur laquelle est posée une couche filtrante comprenant des ouvertures de taille et densité correspondant à la perméabilité désirée selon le type et taille des particules à traiter.

8. Etuve (1) selon l'une quelconque des revendications précédentes, dans laquelle les premier et second moyens de répartition (2a, 2b) des particules sur les premier et second plateaux (1a, 1b), respectivement, comprennent chacun au moins une vis d'Archimède s'étendant le long d'un rayon des premier et second plateaux (1a, 1b), respectivement, ladite au moins une vis d'Archimède étant enfermée dans une enceinte munie d'une ou plusieurs ouvertures s'étendant le long dudit rayon des plateaux (1a, 1b).

9. Etuve (1) selon l'une quelconque des revendications précédentes, dans laquelle le moyen de récupération (3a) du premier plateau (1a) comprend au moins une vis d'Archimède s'étendant le long d'un rayon dudit plateau qui est enfermée dans une enceinte munie d'une ou plusieurs ouvertures s'étendant le long dudit rayon du premier plateau (1a), lesdites ouvertures étant reliées à un racleur ou brosse apte à récolter et diriger les particules amenées par la rotation du plateau vers la vis d'Archimède.

10. Etuve (1) selon l'une quelconque des revendications précédentes, comprenant de plus un second moyen de récupération (3b) des particules réparties sur le second plateau (1b) après une rotation d'un angle donné de celui-ci, le dit second moyen de récupération étant situé en aval du, préférablement adjacent au second moyen de répartition (2b), ledit moyen de récupération permettant de récupérer les particules sur le second plateau et de les transférer vers l'extérieur de l'enceinte.

11. Etuve (1) selon la revendication 10, dans lequel le second moyen de récupération (3b) du second plateau (1b) comprend au moins une vis d'Archimède s'étendant le long d'un rayon dudit plateau qui est enfermée dans une enceinte munie d'une ou plusieurs ouvertures s'étendant le long dudit rayon du second plateau (1b), lesdites ouvertures étant reliées à un racleur ou brosse apte à récolter et diriger les particules amenées par la rotation du plateau vers la vis d'Archimède.

12. Etuve selon l'une quelconque des revendications précédentes, dans laquelle l'axe vertical, Z, est centré sur le conduit de chauffage (6) qui forme une enceinte centrale essentiellement cylindrique creuse dont la paroi s'étend au moins du premier plateau (1a) au second plateau (1b), ladite enceinte contenant la soufflerie et la station de chauffage.

13. Etuve (1) selon l'une quelconque des revendications précédentes, comprenant un plancher statique situé en dessous du plateau inférieur situé le plus bas dudit axe vertical, Z, ledit plancher comprenant une ouverture d'évacuation des particules les plus fines qui se seraient déposées sur le plancher, ladite étuve comprenant de plus un racleur fixé de manière solidaire au plateau inférieur situé le plus bas et apte à suivre le mouvement de rotation de celui-ci pour pousser les particules déposées sur le plancher vers ladite ouverture d'évacuation.

14. Etuve (1) selon l'une quelconque des revendications précédentes, dans lequel le premier moyen de répartition (2a) desdites particules sur le premier plateau (1a) est relié en amont à une source (11) desdites particules, de préférence un silo (11), lesdites particules comprenant de manière préférée des déchets ou sous-produits:
• de bois de scieries ou de bois de matériaux de construction;
• de papier ou cartons,
dans lesquels lesdits déchets ou sous-produits sont sous forme de poudre, de sciure, de granulés, de copeaux, de plaquette, de pellets, de tourteaux, et ou les particules ont de préférence une plus grande taille moyenne comprise entre 1 et 150 mm, de préférence entre 5 et 50 mm.

15. Procédé de traitement de particules organiques d'origine végétale pour l'élimination d'organismes nuisibles présentant des risques phytosanitaires, ledit procédé comprenant l'utilisation d'une étuve selon l'une quelconque des revendications précédentes pour effectuer les étapes suivantes,
(a) Former un flux de gaz chaud (52) en soufflant à l'aide de la soufflerie (5) de ladite étuve (1) un gaz froid (51) à travers la station de chauffage (7) de l'étuve, et diriger le flux de gaz chaud (52) ainsi formé à une première température, T1, et une première humidité relative, RH1, suivant un flux sensiblement parallèle à l'axe Z, passant d'abord à travers le premier plateau (1a) avant de passer directement après à travers le second plateau (1b) ;
(b) Répartir les particules (200) à traiter sur le premier plateau (1a) circulaire traversé en premier par le flux de gaz chaud (52) et faire tourner le premier plateau autour de l'axe vertical, Z, à la première vitesse de rotation, v1, afin que les particules (20a) réparties sur le premier plateau atteignent une température de traitement, Tt, après une rotation d'un premier angle, θ, donné,
(c) Après rotation du premier plateau d'un angle, θ, donné, récupérer les particules ayant la température de traitement, Tt, dudit premier plateau et les transférer vers et répartir sur,
(d) le second plateau (1b) circulaire qui est traversé par un flux de gaz refroidi (53) ayant une seconde température, T2 ≥ Tt, et une seconde humidité relative, RH2, après avoir traversé le premier plateau, et faire tourner le second plateau autour de l'axe vertical, Z, à la seconde vitesse de rotation, v2, afin de maintenir les particules à la température de traitement, Tt, pendant un temps, t1,
(e) Après rotation du second plateau d'un second angle, θ, donné, récupérer les particules (20b) ayant la température de traitement, Tt, dudit second plateau et les transférer hors de l'étuve, et
(f) Diriger le flux de gaz froid (54) ayant une troisième température, T3 < T2, et une troisième humidité relative, RH3 > RH2, après avoir traversé le second plateau vers la soufflerie et répéter les étapes (a) à (f).

## Patentansprüche

1. Ofen (1) zur Beseitigung von schädlichen Organismen, die phytosanitäre Risiken aufweisen und in Material pflanzlichen Ursprungs in Form von Partikeln vorkommen, wobei der Ofen umfasst:
(a) ein Gehäuse (8), das eine im Wesentlichen zylindrische Wand umfasst, die sich entlang einer vertikalen Achse Z erstreckt,
(b) eine kreisrunde erste Scheibe (1a), die im Wesentlichen senkrecht zur vertikalen Achse Z an der Wand des Gehäuses (8) montiert ist und dazu eingerichtet ist, mit einer ersten Drehzahl v1 in einer ersten Richtung um die vertikale Achse Z zu drehen, wobei die Fläche der Scheibe gelocht ist und für Luft, Wasserdampf und Wasser durchlässig ist,
(c) eine kreisrunde zweite Scheibe (1b), die in einem gewissen Abstand von der ersten Scheibe im Wesentlichen senkrecht zur vertikalen Achse Z an der Wand des Gehäuses (8) montiert ist und dazu eingerichtet ist, mit einer zweiten Drehzahl v2 um die vertikale Achse Z zu drehen, bevorzugt in die zur ersten Scheibe entgegengesetzte Drehrichtung, wobei die Fläche der Scheibe gelocht ist und für Luft, Wasserdampf und Wasser durchlässig ist,
(d) ein erstes Verteilungsmittel (2a) zum Verteilen der Partikel, das geeignet ist, die Partikel vor dem Trocknen entlang eines Radius der ersten Scheibe (1a) zu verteilen,
(e) ein erstes Rückgewinnungsmittel (3a) zum Rückgewinnen der Partikel (20a), die auf der ersten Scheibe (1a) verteilt sind, nach deren Drehung um einen gegebenen Winkel, wobei das erste Rückgewinnungsmittel nachgelagert zum und bevorzugt benachbart zum ersten Verteilungsmittel (2a) angeordnet ist,
(f) ein Transfermittel (4a) zum Transferieren der Partikel, die auf der ersten Scheibe (1a) vom ersten Rückgewinnungsmittel (3a) gesammelt wurden, zu einem zweiten Verteilungsmittel (2b), das geeignet ist, die Partikel (20t) entlang eines Radius der zweiten Scheibe (1b) zu verteilen, und
(g) ein Gasblasmittel, das einen geschlossenen Gaskreislauf bildet, umfassend:
• ein Gebläse (5), um einem Gasstrom (51) eine Geschwindigkeit zu verleihen und ihn zu führen zu
• einer Heizstation (7), um einen heißen Gasstrom (52) zu bilden, der eine Ausgangstemperatur T0 und eine relative Ausgangsfeuchte RH0 hat, und ihn anschließend zu führen zu
• einem vorgelagerten Deflektor, der den heißen Gasstrom gemäß einem im Wesentlichen parallel zur Achse Z verlaufenden Strom umlenkt, der eine erste Temperatur T1 und eine erste relative Feuchte RH1 hat, zunächst durch die gelochte Fläche der ersten Scheibe (1a) durchtritt, wo er Wärmeenergie verliert und aus der ein abgekühlter Gasstrom (53) hervorgeht, der eine zweite Temperatur T2 und eine zweite relative Feuchte RH2 hat, um anschließend direkt danach durch die gelochte Fläche der zweiten Scheibe (1b) durchzutreten, wo er weiter Wärmeenergie verliert und aus der ein kalter Gasstrom (54) hervorgeht, der eine dritte Temperatur T3 und eine dritte relative Feuchte RH3 hat, um anschließend zu erreichen
• einen nachgelagerten Deflektor, der den kalten Gasstrom (54) zu dem Gebläse umlenkt, und den Gaskreislauf erneut zu beginnen.

2. Ofen (1) nach Anspruch 1, bei dem die erste Scheibe (1a) über der zweiten Scheibe (1b) angeordnet ist und bei dem das heiße Gas von oben nach unten strömt und bevorzugt Heißluft ist.

3. Ofen (1) nach Anspruch 1, bei dem die erste Scheibe (1a) unter der zweiten Scheibe (1b) angeordnet ist und bei dem das heiße Gas von unten nach oben strömt und bevorzugt Heißluft ist.

4. Ofen (1) nach einem der vorhergehenden Ansprüche, umfassend einen Controller, der dazu ausgestaltet ist zu kontrollieren, dass die erste Drehzahl v1 der ersten Scheibe größer als die zweite Drehzahl v2 der zweiten Scheibe ist, mit v2 = 1/k • v1, worin |k| ≥1 ist und der absolute Wert von k bevorzugt zwischen 1 und 5, bevorzugt zwischen 2 und 4 beträgt, noch bevorzugter |k| = 3 ist, und worin v2 bevorzugt zwischen 0,5 und 1,2 Umdrehungen pro Stunde beträgt.

5. Ofen (1) nach einem der vorhergehenden Ansprüche, umfassend einen Controller, der dazu ausgestaltet ist zu kontrollieren, dass die erste Temperatur T1 des heißen Gasstroms (52) zwischen 75 und 120 °C, bevorzugt zwischen 85 und 100 °C, noch bevorzugter zwischen 90 und 95 °C beträgt und die erste relative Feuchte RH1 des heißen Gasstroms zwischen 15 und 60 % beträgt, bevorzugt RH1 ≥ 20 % ist.

6. Ofen (1) nach einem der vorhergehenden Ansprüche, umfassend einen Controller, der dazu ausgestaltet ist zu kontrollieren, dass
• die zweite Temperatur T2 des abgekühlten Gasstroms (53) zwischen 60 und 80 °C, bevorzugt zwischen 65 und 70 °C beträgt und die zweite relative Feuchte RH2 des abgekühlten Gasstroms zwischen 60 und 90 %, bevorzugt zwischen 75 und 85 % beträgt, und dass
• die dritte Temperatur T3 des kalten Gasstroms (54) zwischen 55 und 65 °C, bevorzugt zwischen 58 und 62 °C beträgt und die dritte relative Feuchte RH3 des abgekühlten Gasstroms zwischen 80 und 100 %, bevorzugt zwischen 95 und 99 % beträgt.

7. Ofen (1) nach einem der vorhergehenden Ansprüche, bei dem die ersten und zweiten Scheiben (1a, 1b) eine selbsttragende starre Struktur mit hoher Durchlässigkeit von der Art eines Gitterrostes umfassen, auf die eine filternde Schicht aufgebracht ist, die Öffnungen mit einer Größe und Dichte umfasst, die der gewünschten Durchlässigkeit gemäß der Art und der Größe der zu behandelnden Partikel entsprechen.

8. Ofen (1) nach einem der vorhergehenden Ansprüche, bei dem die ersten und zweiten Verteilungsmittel (2a, 2b) zum Verteilen der Partikel auf den ersten beziehungsweise zweiten Scheiben (1a, 1b) jeweils mindestens eine archimedische Schraube umfassen, die sich entlang eines Radius der ersten beziehungsweise zweiten Scheiben (1a, 1b) erstreckt, wobei die mindestens eine archimedische Schraube in einem Gehäuse eingeschlossen ist, das mit einer oder mehreren Öffnungen versehen ist, die sich entlang des Radius der Scheiben (1a, 1b) erstrecken.

9. Ofen (1) nach einem der vorhergehenden Ansprüche, bei dem das Rückgewinnungsmittel (3a) der ersten Scheibe (1a) mindestens eine archimedische Schraube umfasst, die sich entlang eines Radius der Scheibe erstreckt und die in einem Gehäuse eingeschlossen ist, das mit einer oder mehreren Öffnungen versehen ist, die sich entlang des Radius der ersten Scheibe (1a) erstrecken, wobei die Öffnungen mit einem Abstreifer oder einer Bürste verbunden sind, der oder die geeignet ist, die durch die Drehung der Scheibe zugeführten Partikel zu sammeln und zur archimedischen Schraube zu führen.

10. Ofen (1) nach einem der vorhergehenden Ansprüche, umfassend zudem ein zweites Rückgewinnungsmittel (3b) zum Rückgewinnen der auf der zweiten Scheibe (1b) verteilten Partikel nach deren Drehung um einen gegebenen Winkel, wobei das zweite Rückgewinnungsmittel nachgelagert zum, bevorzugt benachbart zum zweiten Verteilungsmittel (2b) angeordnet ist, wobei das Rückgewinnungsmittel es ermöglicht, die Partikel auf der zweiten Scheibe zurückzugewinnen und sie nach außerhalb des Gehäuses zu transferieren.

11. Ofen (1) nach Anspruch 10, bei dem das zweite Rückgewinnungsmittel (3b) der zweiten Scheibe (1b) mindestens eine archimedische Schraube umfasst, die sich entlang eines Radius der Scheibe erstreckt und die in einem Gehäuse eingeschlossen ist, das mit einer oder mehreren Öffnungen versehen ist, die sich entlang des Radius der zweiten Scheibe (1b) erstrecken, wobei die Öffnungen mit einem Abstreifer oder einer Bürste verbunden sind, der oder die geeignet ist, die durch die Drehung der Scheibe zugeführten Partikel zu sammeln und zur archimedischen Schraube zu führen.

12. Ofen nach einem der vorhergehenden Ansprüche, bei dem die vertikale Achse Z auf den Heizkanal (6) zentriert ist, der eine im Wesentlichen zylindrisches hohles zentrales Gehäuse bildet, dessen Wand sich mindestens von der ersten Scheibe (1a) zur zweiten Scheibe (1b) erstreckt, wobei das Gehäuse das Gebläse und die Heizstation enthält.

13. Ofen (1) nach einem der vorhergehenden Ansprüche, umfassend einen statischen Boden, der unter der unteren Scheibe angeordnet ist, die am weitesten unten an der vertikalen Achse Z angeordnet ist, wobei der Boden eine Austragöffnung zum Austragen der feinsten Partikel umfasst, die auf dem Boden abgesetzt werden sollten, wobei der Ofen zudem einen Abstreifer umfasst, der fest an der am weitesten unten angeordneten Scheibe befestigt ist und geeignet ist, deren Drehbewegung zu folgen, um die auf dem Boden abgesetzten Partikel zur Austragöffnung zu schieben.

14. Ofen (1) nach einem der vorhergehenden Ansprüche, bei dem das erste Verteilungsmittel (2a) zum Verteilen der Partikel auf der ersten Scheibe (1a) vorgelagert mit einer Quelle (11) für die Partikel, bevorzugt einem Silo (11), verbunden ist, wobei die Partikel bevorzugt Abfälle oder Nebenprodukte umfassen:
• von Holz aus Sägewerken oder von Holz aus Baustoffen;
• von Papier oder Pappen,
wobei die Abfälle oder Nebenprodukte in Form von Pulver, Sägemehl, Spänen, Schnitzeln, Pellets, Presskuchen vorliegen und die Partikel bevorzugt eine durchschnittliche größte Größe zwischen 1 und 150 mm, bevorzugt zwischen 5 und 50 mm haben.

15. Verfahren zur Behandlung von organischen Partikeln pflanzlichen Ursprungs zur Beseitigung von schädlichen Organismen, die phytosanitäre Risiken aufweisen, wobei das Verfahren die Verwendung eines Ofens nach einem der vorhergehenden Ansprüche umfasst, um die folgenden Schritte durchzuführen:
(a) Bilden eines heißen Gasstroms (52), indem mithilfe des Gebläses (5) des Ofens (1) ein kaltes Gas (51) durch die Heizstation (7) des Ofens geblasen wird, und Führen des so gebildeten heißen Gasstroms (52) mit einer ersten Temperatur T1 und einer ersten relativen Feuchte RH1 gemäß einem im Wesentlichen parallel zur Achse Z verlaufenden Strom, der zunächst durch die erste Scheibe (1a) durchtritt, bevor er direkt danach durch die zweite Scheibe (1b) durchtritt;
(b) Verteilen der zu behandelnden Partikel (200) auf der kreisrunden ersten Scheibe (1a), die zuerst von dem heißen Gasstrom (52) durchströmt wird, und Drehen der ersten Scheibe um die vertikale Achse Z mit der ersten Drehzahl v1, damit die Partikel (20a), die auf der ersten Scheibe verteilt sind, eine Behandlungstemperatur Tt nach einer Drehung um einen gegebenen ersten Winkel θ erreichen,
(c) Nach Drehung der ersten Scheibe um einen gegebenen Winkel θ, Rückgewinnen der Partikel, die die Behandlungstemperatur Tt haben, von der ersten Scheibe und Transferieren von diesen zu der und Verteilen von diesen auf der
(d) kreisrunden zweiten Scheibe (1b), die von einem abgekühlten Gasstrom (53) durchströmt wird, der eine zweite Temperatur T2 ≥ Tt und eine zweite relative Feuchte RH2 hat, nachdem er die erste Scheibe durchströmt hat, und Drehen der zweiten Scheibe um die vertikale Achse Z mit der zweiten Drehzahl v2, um die Partikel während einer Zeit t1 auf der Behandlungstemperatur Tt zu halten,
(e) Nach Drehung der zweiten Scheibe um einen gegebenen zweiten Winkel θ, Rückgewinnen der Partikel (20b), die die Behandlungstemperatur Tt haben, von der zweiten Scheibe und Transferieren von diesen aus dem Ofen heraus und
(f) Führen des kalten Gasstroms (54), der eine dritte Temperatur T3 < T2 und eine dritte relative Feuchte RH3 > RH2 hat, nachdem er die zweite Scheibe durchströmt hat, zu dem Gebläse und Wiederholen der Schritte (a) bis (f).

## Claims

1. Oven (1) for the elimination of harmful organisms that present phytosanitary risks present in materials of plant origin in the form of particles, said oven comprising,
(a) an enclosure (8) comprising an essentially cylindrical wall extending along a vertical axis, Z,
(b) a first circular plate (1a) mounted on the wall of said enclosure (8) substantially normal to the vertical axis, Z, and arranged to rotate at a first speed of rotation, v1, in a first direction about the vertical axis, Z, the surface of said plate being perforated, and permeable to air, to water vapour and to water,
(c) a second circular plate (1b) mounted at a certain distance from the first plate on the wall of said enclosure (8) substantially normal to the vertical axis, Z, and arranged to rotate at a second speed of rotation, v2, about said vertical axis, Z, preferably in the reverse direction of rotation to the first plate, the surface of said plate being perforated and permeable to air, to water vapour and to water,
(d) a first distribution means (2a) for distributing said particles capable of distributing said particles before baking along a radius of the first plate (1a),
(e) a first recovery means (3a) for recovering the particles (20a) distributed on the first plate (1a) after a rotation by a given angle thereof, said first recovery means being situated downstream of, and preferably adjacent to, the first distribution means (2a),
(f) a transfer means (4a) for transferring the particles collected from the first plate (1a) by the first recovery means (3a) to a second distribution means (2b) capable of distributing said particles (20t) along a radius of the second plate (1b), and
(g) a gas blowing means forming a closed gas cycle, comprising:
• a blower (5) for imparting a velocity on a flow of gas (51) and directing it toward,
• a heating station (7) to form a hot gas flow (52) having an initial temperature, T0, and an initial relative humidity, RH0, and then directing it toward,
• an upstream baffle, deflecting the hot gas flow as a flow substantially parallel to the axis Z, having a first temperature, T1, and a first relative humidity, RH1, passing first of all through the perforated surface of the first plate (1a), where it loses calorific energy and from where a cooled gas flow (53) emerges having a second temperature, T2, and a second relative humidity, RH2, to then pass directly afterward through the perforated surface of the second plate (1b), where it loses more calorific energy and from where a cold gas flow (54) emerges having a third temperature, T3, and a third relative humidity, RH3, to then reach,
• a downstream baffle deflecting the cold gas flow (54) toward the blower and recommence the gas cycle.

2. Oven (1) according to Claim 1, wherein the first plate (1a) is situated above the second plate (1b) and in which the hot gas circulates from top to bottom and is preferably hot air.

3. Oven (1) according to Claim 1, wherein the first plate (1a) is situated below the second plate (1b) and in which the hot gas circulates from bottom to top and is preferably hot air.

4. Oven (1) according to any one of the preceding claims, comprising a controller configured to check that the first speed of rotation, v1, of the first plate is greater than the second speed of rotation, v2, of the second plate, with v2 = 1/k · v1, wherein, |k| ≥ 1, and the absolute value of k is preferably between 1 and 5, preferably between 2 and 4, more preferably, |k| = 3, and wherein v2 is preferably between 0.5 and 1.2 revolutions per hour.

5. Oven (1) according to any one of the preceding claims, comprising a controller configured to check that the first temperature, T1, of the hot gas flow (52) is between 75 and 120°C, preferably between 85 and 100°C, more preferably between 90 and 95°C, and the first relative humidity, RH1, of said hot gas flow is between 15 and 60%, preferably RH1 ≥ 20%.

6. Oven (1) according to any one of the preceding claims, comprising a controller configured to check that,
• the second temperature, T2, of the cooled gas flow (53) is between 60 and 80°C, preferably between 65 and 70°C, and the second relative humidity, RH2, of said cooled gas flow is between 60 and 90%, preferably between 75 and 85%, and that,
• the third temperature, T3, of the cold gas flow (54) is between 55 and 65°C, preferably between 58 and 62°C, and the third relative humidity, RH3, of said cooled gas flow is between 80 and 100%, preferably between 95 and 99%.

7. Oven (1) according to any one of the preceding claims, wherein the first and second plates (1a, 1b) comprise a self-supporting rigid structure with high permeability of grating type, on which is placed a filtering layer comprising openings of a size and density corresponding to the permeability desired according to the type and size of the particles to be treated.

8. Oven (1) according to any one of the preceding claims, wherein the first and second distribution means (2a, 2b) for distributing the particles on the first and second plates (1a, 1b), respectively, each comprise at least one Archimedes screw extending along a radius of the first and second plates (1a, 1b), respectively, said at least one Archimedes screw being enclosed in an enclosure provided with one or more openings extending along said radius of the plates (1a, 1b).

9. Oven (1) according to any one of the preceding claims, wherein the recovery means (3a) of the first plate (1a) comprises at least one Archimedes screw extending along the radius of said plate which is enclosed in an enclosure provided with one or more openings extending along said radius of the first plate (1a), said openings being linked to a scraper or brush capable of collecting and directing the particles brought by the rotation of the plate to the Archimedes screw.

10. Oven (1) according to any one of the preceding claims, also comprising a second recovery means (3b) for recovering the particles distributed on the second plate (1b) after a rotation by a given angle thereof, said second recovery means being situated downstream of, preferably adjacent to, the second distribution means (2b), said recovery means making it possible to recover the particles on the second plate and to transfer them out of the enclosure.

11. Oven (1) according to Claim 10, wherein the second recovery means (3b) of the second plate (1b) comprises at least one Archimedes screw extending along a radius of said plate which is enclosed in an enclosure provided with one or more openings extending along said radius of the second plate (1b), said openings being linked to a scraper or brush capable of collecting and directing the particles brought by the rotation of the plate to the Archimedes screw.

12. Oven according to any one of the preceding claims, wherein the vertical axis, Z, is centred on the heating duct (6) which forms an essentially cylindrical hollow central enclosure whose wall extends at least from the first plate (1a) to the second plate (1b), said enclosure containing the blower and the heating station.

13. Oven (1) according to any one of the preceding claims, comprising a static floor situated below the lower plate situated lowest on said vertical axis, Z, said floor comprising an opening for discharging the finest particles which would be deposited on the floor, said oven also comprising a scraper fixed securely to the lower plate situated lowest and capable of following the rotational movement thereof to push the particles deposited on the floor towards said discharge opening.

14. Oven (1) according to any one of the preceding claims, wherein the first distribution means (2a) for distributing said particles on the first plate (1a) is linked upstream to a source (11) of said particles, preferably a silo (11), said particles preferably comprising waste or by-products:
• of wood from sawmills or of construction material wood;
• of paper or cardboard,
wherein said waste or by-products are in the form of powder, sawdust, flakes, chips, wafers, pellets, cakes, and/or the particles preferably have a larger average size of between 1 and 150 mm, preferably between 5 and 50 mm.

15. Method for treating organic particles of plant origin for the elimination of harmful organisms presenting phytosanitary risks, said method comprising the use of an oven according to any one of the preceding claims to perform the following steps,
(a) forming a hot gas flow (52) by blowing, using the blower (5) of said oven (1), a cold gas (51) through the heating station (7) of the oven, and directing the hot gas flow (52) thus formed at a first temperature, T1, and a first relative humidity, RH1, as a flow substantially parallel to the axis Z, passing first of all through the first plate (1a) before passing directly afterward through the second plate (1b) ;
(b) distributing the particles (200) to be treated on the first circular plate (1a) passed through first by the hot gas flow (52) and rotating the first plate about the vertical axis, Z, at the first speed of rotation, v1, in order for the particles (20a) distributed on the first plate to reach a treatment temperature, Tt, after a rotation by a given first angle, θ,
(c) after rotation of the first plate by a given angle, θ, recovering the particles having the treatment temperature, Tt, from said first plate and transferring them to and distributing them on,
(d) the second circular plate (1b) which is passed through by a cooled gas flow (53) having a second temperature, T2 ≥ Tt, and a second relative humidity, RH2, after having passed through the first plate, and rotating the second plate about the vertical axis, Z, at the second speed of rotation, v2, in order to keep the particles at the treatment temperature, Tt, for a time, t1,
(e) after rotation of the second plate by a given second angle, θ, recovering the particles (20b) having the treatment temperature, Tt, from said second plate and transferring them out of the oven, and
(f) directing the cold gas flow (54) having a third temperature, T3 < T2, and a third relative humidity, RH3 > RH2, after having passed through the second plate to the blower and repeating the steps (a) to (f).
